# EUROPÄISCHE PATENTSCHRIFT

(11) **EP 0 670 836 B1**
(45) Veröffentlichungstag und Bekanntmachung des Hinweises auf die Patenterteilung: **04.10.2001**
(21) Anmeldenummer: 94901802.2
(22) Anmeldetag: 15.11.1993
(51) Int. Cl.: C07D 417/04, A01N 43/88, C07D 285/08

(54) **3-PYRIDYL-1,2,4-THIADIAZOLE ALS PESTIZIDE**
PYRIDYL-1,2,4-THIADIAZOLES AS PEST-CONTROL AGENTS
3-PYRIDYL-1,2,4-THIADIAZOLES UTILISES COMME PESTICIDES

(30) Priorität: 26.11.1992 DE 4239727
(43) Veröffentlichungstag der Anmeldung: 13.09.1995
(73) Patentinhaber: BAYER AG, 51368 Leverkusen (DE)
(72) Erfinder: HEINEMANN, Ulrich, D-42799 Leichlingen (DE); TIEMANN, Ralf, D-51377 Leverkusen (DE); DEHNE, Heinz-Wilhelm, D-40789 Monheim (DE)
(86) Internationale Anmeldenummer: EP9303198
(87) Internationale Veröffentlichungsnummer: WO9412496

(56) Entgegenhaltungen:
- EP-A- 0 116 515
- EP-A- 0 273 534
- EP-A- 0 285 565
- EP-A- 0 486 798
- WO-A-92/16527
- FR-A- 1 518 150
- US-A- 3 736 328
- US-A- 3 770 749

## Beschreibung

Die vorliegende Erfindung betrifft neue Pyridyl-1,2,4-thiadiazole, ein Verfahren zu ihrer Herstellung und ihre Verwendung als Schädlingsbekämpfungsmittel.

Es ist bereits bekannt geworden, daß bestimmte substituierte Heterocyclen fungizide Eigenschaften besitzen (vgl. DE-OS 4 033 412). So lassen sich zum Beispiel 2-(3-Trifluormethyl-phenyl)-5-methylsulfonyl-1,3,4-oxadiazol und 2-(4-Fluorphenyl)-5-methylsulfonyl-1,3,4-oxadiazol zur Bekämpfung von Pilzen verwenden. Die Wirksamkeit dieser Stoffe ist aber insbesondere bei niedrigen Aufwandmengen nicht in allen Anwendungsgebieten völlig zufriedenstellend. In WO 92/16527 werden in 2-Stellung substituierte Pyridin-Derivate beschrieben, die eine bakterizide Wirkung zeigen.

Es wurden nun neue Pyridyl-1,2,4-thiadiazole der Formel 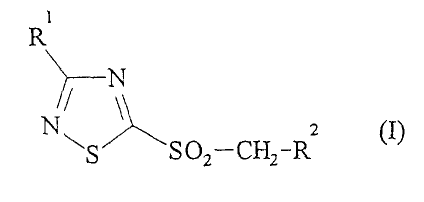 in welcher
- R¹: für 2-Pyridyl, 3-Pyridyl oder 4-Pyridyl steht, wobei jeder dieser Reste einfach bis vierfach, gleichartig oder verschieden substituiert sein kann durch Halogen, Cyano, Nitro, jeweils geradkettiges oder verzweigtes Alkyl, Alkoxy oder Alkylsulfonyl mit jeweils 1 bis 6 Kohlenstoffatomen, jeweils geradkettiges oder verzweigtes Halogenalkyl, Halogenalkoxy oder Halogenalkylsulfonyl mit jeweils 1 bis 6 Kohlenstoffatomen und 1 bis 13 gleichen oder verschiedenen Halogenatomen, geradkettiges oder verzweigtes Alkoxycarbonyl mit 1 bis 6 Kohlenstoffatomen in der Alkoxygruppe, geradkettiges oder verzweigtes
Alkoximinoalkyl mit 1 bis 6 Kohlenstoffatomen im Alkoxyteil und 1 bis 6 Kohlenstoffatomen im Alkylteil sowie durch Phenyl, welches seinerseits einfach bis vierfach, gleichartig oder verschieden substituiert sein kann durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen, und
- R²: für Aryl mit 6 bis 10 Kohlenstoffatomen steht, wobei jeder dieser Reste einfach bis fünffach, gleichartig oder verschieden substituiert sein kann durch Halogen, Cyano, Nitro, jeweils geradkettiges oder verzweigtes Alkyl, Alkoxy oder Alkylsulfonyl mit jeweils 1 bis 6 Kohlenstoffatomen, jeweils geradkettiges oder verzweigtes Halogenalkyl, Halogenalkoxy oder Halogenalkylsulfonyl mit jeweils 1 bis 6 Kohlenstoffatomen und 1 bis 13 gleichen oder verschiedenen Halogenatomen, geradkettiges oder verzweigtes Alkoxycarbonyl mit 1 bis 6 Kohlenstoffatomen in der Alkoxygruppe, geradkettiges oder verzweigtes Alkoximinoalkyl mit 1 bis 6 Kohlenstoffatomen im Alkoxyteil und 1 bis 6 Kohlenstoffatomen im Alkylteil sowie durch Phenyl, welches seinerseits einfach bis fünffach, gleichartig oder verschieden substituiert sein kann durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen.

Weiterhin wurde gefunden, daß man Pyridyl-1,2,4-thiadiazole der Formel (I) sowie deren Säureadditions-Salze und Metallsalz-Komplexe erhält, wenn man 5-Arylalkylthio-1,2,4-thiadiazole der Formel 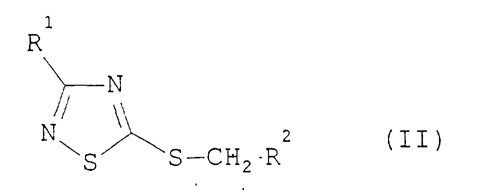 in welcher
- R¹ und R²: die oben angegebenen Bedeutungen haben,
mit einem Oxidationsmittel gegebenenfalls in Gegenwart eines Verdünnungsmittels und gegebenenfalls in Gegenwart eines Säurebindemittels sowie gegebenenfalls in Gegenwart eines Katalysators umsetzt, und gegebenenfalls anschließend an die so erhaltenen Verbindungen der Formel (I) eine Säure oder ein Metallsalz addiert.

Schließlich wurde gefunden, daß die neuen Pyridyl-1,2,4-thiadiazole der Formel (I) sowie deren Säureadditions-Salze und Metallsalz-Komplexe sehr gut zur Schädlingsbekämpfung geeignet sind.

Überraschenderweise besitzen die erfindungsgemäßen Stoffe gegenüber pflanzenschädigenden Mikroorganismen eine erheblich bessere Wirksamkeit als 2-(3-Trifluormethylphenyl)-5-methylsulfonyl-1,3,4-oxadiazol und 2-(4-Fluorphenyl)-5-methylsulfonyl-1,3,4-oxadiazol, welches konstitutionell ähnliche, vorbekannte Wirkstoffe gleicher Wirkungsrichtung sind.
- R¹: steht besonders bevorzugt für 2-Pyridyl, 3-Pyridyl oder 4-Pyridyl, wobei jeder dieser Reste einfach bis vierfach, gleichartig oder verschieden substituiert sein kann durch Fluor, Chlor, Brom, Iod, Cyano, Nitro, jeweils geradkettiges oder verzweigtes Alkyl, Alkoxy oder Alkylsulfonyl mit jeweils 1 bis 4 Kohlenstoffatomen, jeweils geradkettiges oder verzweigtes Halogenalkyl, Halogenalkoxy oder Halogenalkoxysulfonyl mit jeweils 1 bis 4 Kohlenstoffatomen und 1 bis 9 gleichen oder verschiedenen Halogenatomen, wie Fluor, Chlor und/oder Brom, durch geradkettiges oder verzweigtes Alkoxycarbonyl mit 1 bis 4 Kohlenstoffatomen im Alkoxyteil, geradkettiges oder verzweigtes Alkoximinoalkyl mit 1 bis 4 Kohlenstoffatomen im Alkoxyteil und 1 bis 4 Kohlenstoffatomen im Alkylteil und/oder durch Phenyl, welches seinerseits einfach bis dreifach, gleichartig oder verschieden substituiert sein kann durch Fluor, Chlor, Brom, geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 Fluor-, Chlor- und/oder Bromatomen.
- R²: steht besonders bevorzugt für Aryl mit 6 bis 10 Kohlenstoffatomen, wobei jeder dieser Reste einfach bis fünffach, gleichartig oder verschieden substituiert sein kann durch Fluor, Chlor, Brom, Iod, Cyano, Nitro, jeweils geradkettiges oder verzweigtes Alkyl, Alkoxy oder Alkylsulfonyl mit jeweils 1 bis 4 Kohlenstoffatomen, jeweils geradkettiges oder verzweigtes Halogenalkyl, Halogenalkoxy oder Halogenalkylsulfonyl mit jeweils 1 bis 4 Kohlenstoffatomen und 1 bis 9 gleichen oder verschiedenen Halogenatomen, wie Fluor, Chlor und/oder Brom, durch geradkettiges oder verzweigtes Alkoxycarbonyl mit 1 bis 4 Kohlenstoffatomen im Alkoxyteil, geradkettiges oder verzweigtes Alkoximinoalkyl mit 1 bis 4 Kohlenstoffatomen im Alkoxyteil und 1 bis 4 Kohlenstoffatomen im Alkylteil und/oder durch Phenyl, welches seinerseits einfach bis dreifach, gleichartig oder verschieden substituiert sein kann durch Fluor, Chlor, Brom, geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 Fluor-, Chlor- und/oder Bromatomen.
- R¹: steht ganz besonders bevorzugt für 2-Pyridyl, 3-Pyridyl oder 4-Pyridyl, wobei jeder dieser Reste einfach oder zweifach, gleichartig oder verschieden substituiert sein kann durch Fluor, Chlor, Brom, Cyano, Nitro, Methyl, Ethyl, n-Propyl, Isopropyl, Methoxy, Ethoxy, Methylsulfonyl, Trifluormethyl, Trifluormethoxy, Trifluormethylsulfonyl, Methoxycarbonyl, Methoximinomethyl und/ oder durch Phenyl, welches seinerseits einfach oder zweifach, gleichartig oder verschieden substituiert sein kann durch Fluor, Chlor, Methyl, Ethyl, Methoxy, Trifluormethyl oder Trifluormethoxy.
- R²: steht ganz besonders bevorzugt für Phenyl, α-Naphthyl oder β-Naphthyl, wobei jeder dieser Reste einfach bis dreifach, gleichartig oder verschieden substituiert sein kann durch Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Trifluormethyl, Difluormethyl, Trifluormethoxy, Difluormethoxy, Trifluormethylsulfonyl, Methoxycarbonyl, Ethoxycarbonyl, Methoximinomethyl und/oder durch Phenyl, welches seinerseits einfach oder zweifach, gleichartig oder verschieden substituiert sein kann durch Fluor, Chlor, Methyl, Ethyl, Methoxy, Trifluormethyl oder Trifluormethoxy.

Bevorzugte erfindungsgemäße Stoffe sind auch Additionsprodukte aus Säuren und denjenigen Pyridyl-1,2,4-thiadiazolen der Formel (I), in denen R¹ und R² die als bevorzugt angegebenen Bedeutungen haben.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure, Saccharin und Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Pyridyl-1,2,4-thiadiazolen der Formel (I), in denen R¹ und R² die als bevorzugt angegebenen Bedeutungen haben.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Im einzelnen seien außer den bei den Herstellungsbeispielen genannten Verbindungen die folgenden Pyridyl-1,2,4-thiadiazole der Formel (I) genannt:

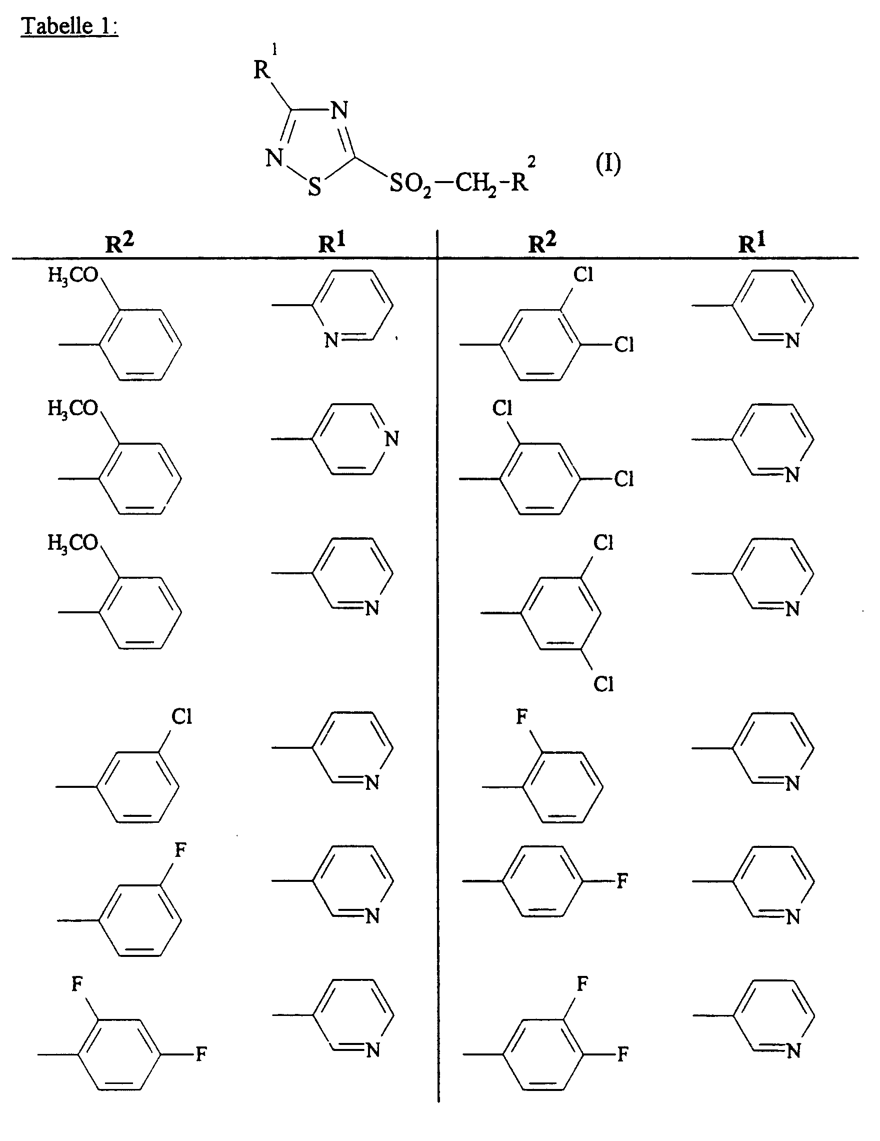

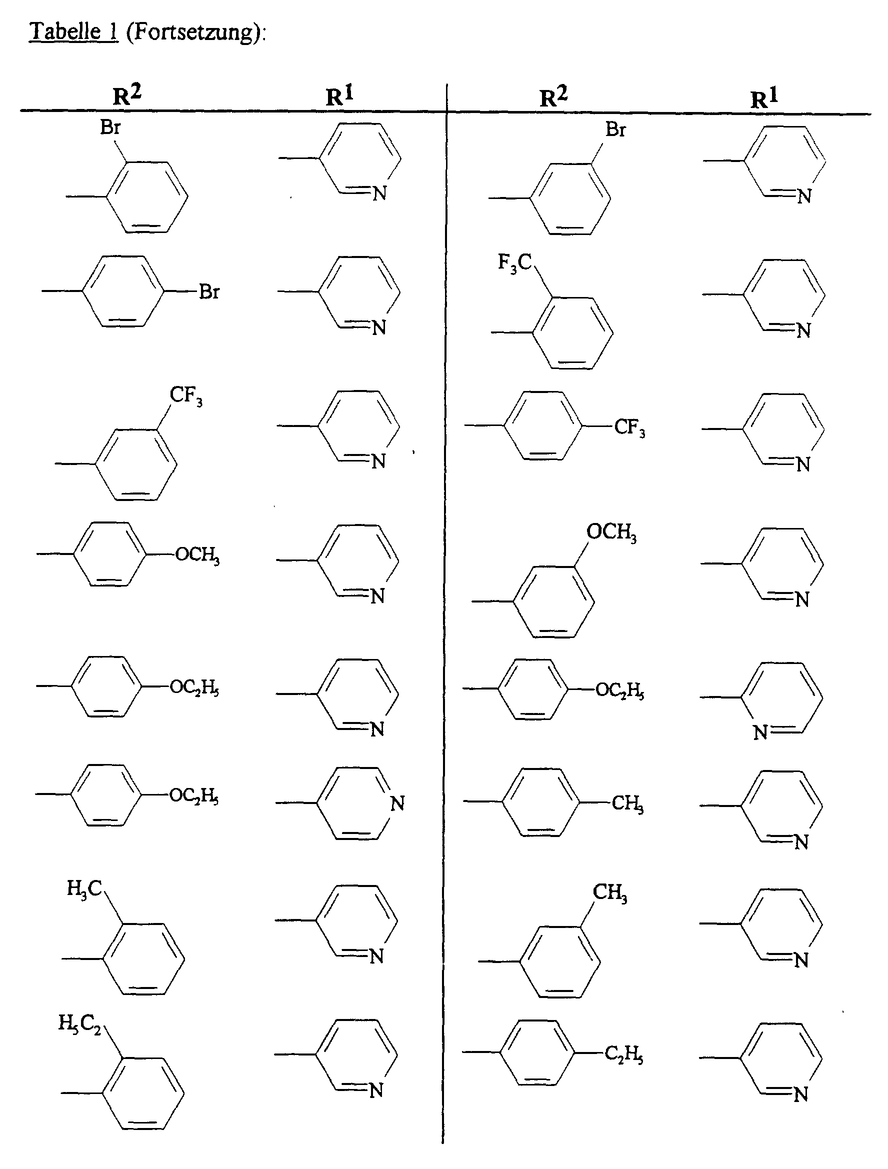

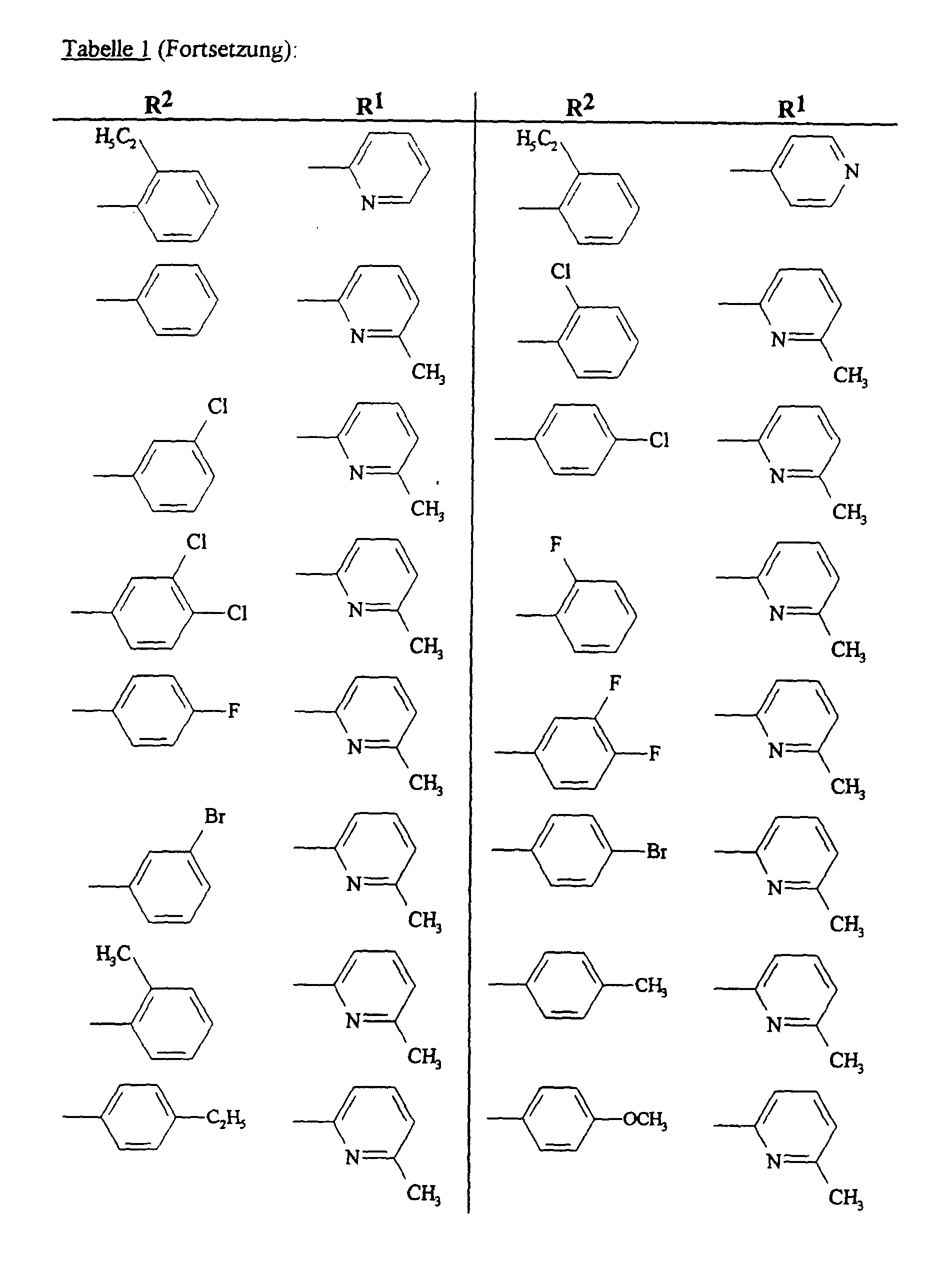

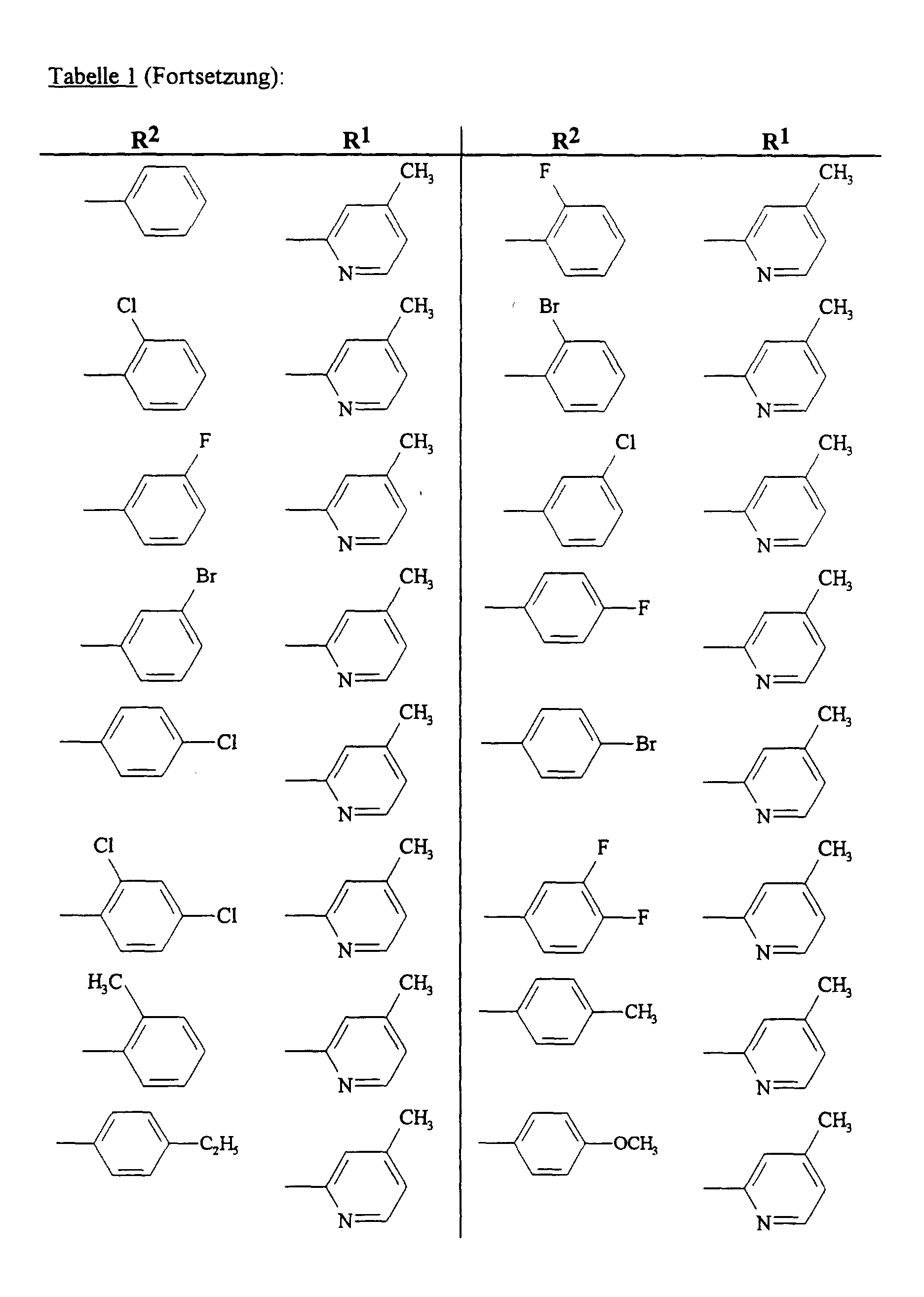

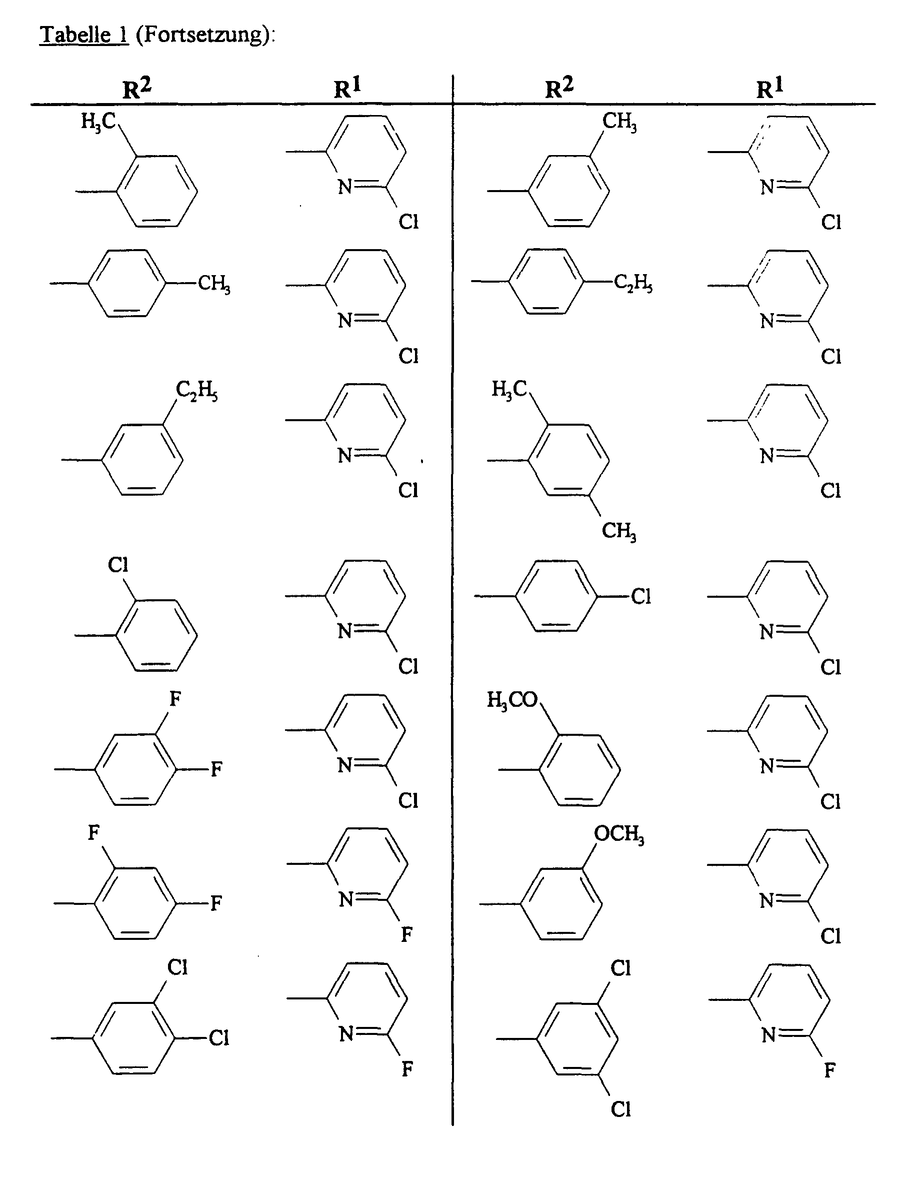

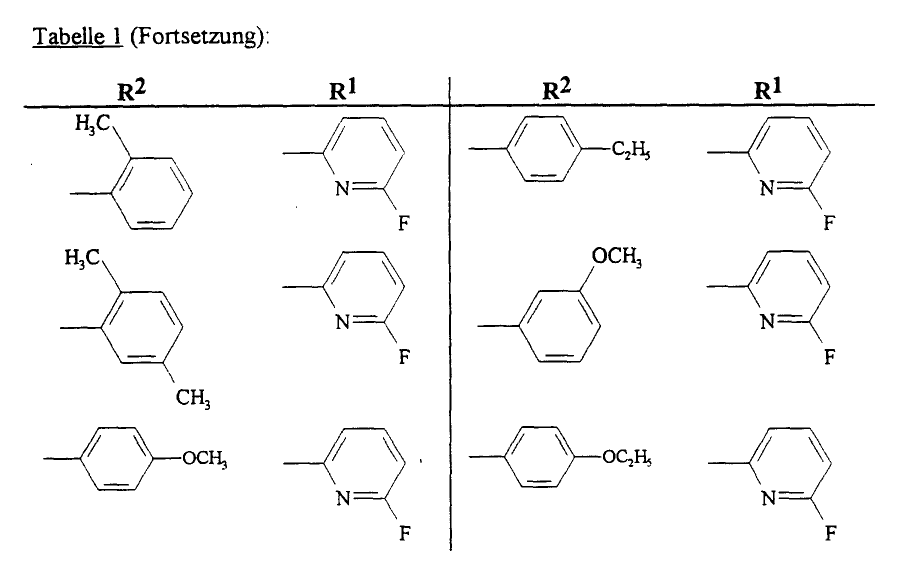

Verwendet man beispielsweise 5-(4-Brombenzylthio)-3-(4-pyridyl)-1,2,4-thiadiazol als Ausgangsverbindung und Kaliumpermanganat als Oxidationsmittel, so läßt sich der Reaktionsablauf des erfindungsgemäßen Verfahrens durch das folgende Formelschema darstellen:

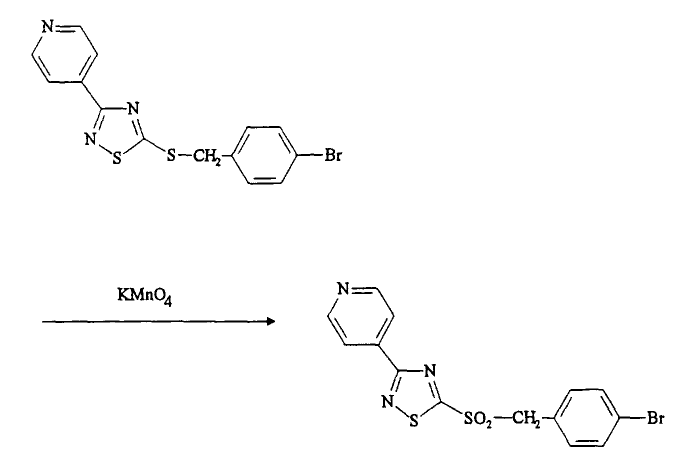

Die zur Durchführung des erfindungsgemäßen Verfahrens als Ausgangsstoffe benötigten 5-Arylalkylthio-1,2,4-thiadiazole sind durch die Formel (II) allgemein definiert. In dieser Formel (II) stehen R¹ und R² vorzugsweise für diejenigen Reste, die bereits im Zusammenhang mit der Beschreibung der erfindungsgemäßen Verbindungen der Formel (I) als bevorzugt für diese Substituenten genannt wurden.

Die 5-Arylalkylthio-1,2,4-thiadiazole der Formel (II) sind noch nicht bekannt und sind ebenfalls Gegenstand der Erfindung. Man erhält sie, wenn man 3-Pyridyl-1,2,4-thiadiazolin-5-thione der Formel 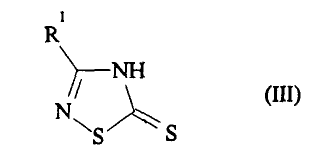 in welcher
- R¹: die oben angegebene Bedeutung hat,
mit Arylalkylhalogeniden der Formel

R²-CH₂-Hal (IV)

in welcher
- R²: die oben angegebene Bedeutung hat und
- Hal: für Halogen, insbesondere für Chlor oder Brom steht,
gegebenenfalls in Gegenwart eines Verdünnungsmittels, wie beispielsweise Acetonitril und gegebenenfalls in Gegenwart eines Reaktionshilfsmittels, wie beispielsweise Kaliumcarbonat, bei Temperaturen zwischen 20°C und 150°C umsetzt. 3-Pyridyl-1,2,4-thiadiazolin-5-thione der Formel (III) sind ebenfalls noch nicht bekannt und sind auch Gegenstand der Erfindung. Man erhält sie, wenn man Pyridylamidinhydrochloride der Formel 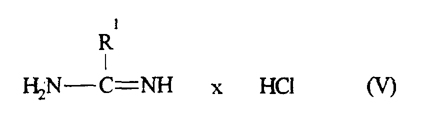 in welcher
- R¹: die oben angegebene Bedeutung hat,
mit Schwefelkohlenstoff in Gegenwart eines Verdünnungsmittels, wie beispielsweise Methanol, und in Gegenwart einen Reaktionshilfsmittels, wie beispielsweise Natriummethylat, sowie in Gegenwart eines Katalysators, wie beispielsweise Schwefel, bei Temperaturen zwischen 20°C und 150°C umsetzt (vergleiche hierzu auch die Herstellungsbeispiele).

Arylalkylhalogenide der Formel (IV) und Pyridylamidinhydrochloride der Formel (V) sind allgemein bekannte Verbindungen der organischen Chemie.

Die Verbindungen der Formel (III) können entweder als 3-Pyridyl-1,2,4-thiadiazolin-5-thione der Formel 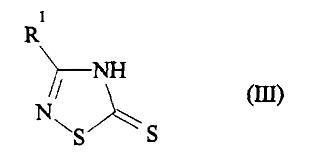 oder in Form ihrer Tautomeren, den 3-Pyridyl-5-mercapto-1,2,4-thiadiazolen der Formel 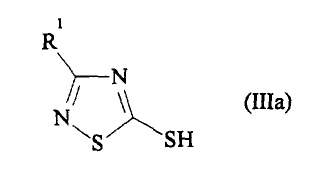 vorliegen. Bei der Umsetzung mit Arylalkylhalogeniden der Formel (IV) reagiert die Mercapto-Form der Formel (IIIa).

Das erfindungsgemäße Verfahren wird in Gegenwart eines geeigneten Oxidationsmittels durchgeführt. Als solche kommen alle üblicherweise für Schwefeloxidationen verwendbaren Oxidationsmittel infrage. Vorzugsweise verwendet man Wasserstoffperoxid oder organische Persäuren, wie beispielsweise Peressigsäure, 4-Nitroperbenzoesäure oder 3-Chlorperbenzoesäure oder auch anorganische Oxidationsmittel, wie Periodsäure, Kaliumpermanganat oder Chromsäure.

Als Verdünnungsmittel zur Durchführung des erfindungsgemäßen Verfahrens kommen in Abhängigkeit vom verwendeten Oxidationsmittel anorganische oder organische Lösungsmittel infrage. Vorzugsweise verwendet man Alkohole, wie Methanol, Ethanol, n- oder i-Propanol, deren Gemische mit Wasser oder reines Wasser; außerdem Säuren wie beispielsweise Essigsäure, Acetanhydrid oder Propionsäure oder dipolar aprotische Lösungsmittel, wie Acetonitril, Aceton, Essigsäureethylester oder Dimethylformamid sowie auch gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Dichlorethan, Chloroform oder Tetrachlorkohlenstoff.

Das erfindungsgemäße Verfahren kann gegebenenfalls auch in Gegenwart eines geeigneten Säurebindemittels durchgeführt werden. Als solche kommen alle üblichen anorganischen oder organischen Basen in Frage. Vorzugsweise verwendbar sind Erdalkalimetall-, Alkalimetall- oder Ammoniumhydroxide, -acetate, -carbonate oder -hydrogencarbonate, wie beispielsweise Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat.

Das erfindungsgemäße Verfahren kann gegebenenfalls auch in Gegenwart eines geeigneten Katalysators durchgeführt werden. Als solche kommen alle üblicherweise für Schwefeloxidationen verwendbaren Katalysatoren in Frage. Vorzugsweise verwendet man Schwermetallkatalysatoren. Beispielhaft genannt seien in diesem Zusammenhang Ammoniummolybdat oder -wolframat.

Die Reaktionstemperaturen können bei der Durchführung des erfindungsgemäßen Verfahrens in einem größeren Bereich variiert werden. Im allgemeinen arbeitet man bei Temperaturen zwischen -30°C und +150°C, vorzugsweise bei Temperaturen zwischen 0°C und 80°C.

Das erfindungsgemäße Verfahren wird üblicherweise unter Normaldruck durchgeführt. Es ist jedoch auch möglich, unter erhöhtem oder vermindertem Druck zu arbeiten.

Zur Durchführung des erfindungsgemäßen Verfahrens setzt man pro Mol an 5-Arylalkylthio-1,2,4-thiadiazol der Formel (II) im allgemeinen 1,8 bis 3,0 Mol, vorzugsweise doppelt molare Mengen an Oxidationsmittel und gegebenenfalls 0,1 bis 3,0 Mol, vorzugsweise 1,0 bis 2,0 Mol an Säurebindemittel sowie gegebenenfalls 0,001 bis 2,0 Mol, vorzugsweise 0,01 bis 1,0 Mol an Katalysator ein. Die Reaktionsdurchführung, Aufarbeitung und Isolierung der Reaktionsprodukte erfolgt nach allgemein bekannten Verfahren (vergl. hierzu auch die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe des Brechungsindex oder der Protonen-Kernresonanzspektroskopie (¹H-NMR).

Die erfindungsgemäßen Pyridyl-1,2,4-thiadiazole der Formel (I) können in Säureadditions-Salze oder Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, Isolieren und gegebenenfalls durch Umkristallisation reinigen.

Die erfindungsgemäßen Wirkstoffe und auch die Arylalkylthio-1,2,4-thiadiazole der Formel (II) weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Krankheiten im Obst- und Gemüseanbau, wie beispielsweise gegen den Erreger des Apfelschorfes (Venturia inaequalis) oder gegen den Erreger des falschen Rebenmehltaues (Plasmopara viticola) oder zur Bekämpfung von Reiskrankheiten, wie beispielsweise gegen den Erreger der Reisfleckenkrankheit (Pyricularia oryzae) eingesetzt werden.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nichtionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden,

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50g je kg Saatgut, vorzugsweise 0,1 bis 10g, benötigt. Bei der Behandlung des Bodens sind Wirkstoffkonzentrationen von 0,00001 bis 0,1 Gew.-%, vorzugsweise 0,0001 bis 0,02 %, am Wirkungsort erforderlich.

Die Herstellung und die Verwendung der erfindungsgemäßen Stoffe geht aus den folgenden Beispielen hervor.

### Herstellungsbeispiele:

### Beispiel 1:

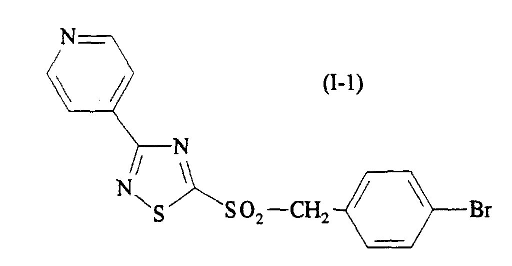

Zu 10,9 g (0,03 Mol) 5-(4-Brombenzylthio)-3-(4-pyridyl)-1,2,4-thiadiazol in 75 ml Eisessig gibt man 9,5 g (0,06 Mol) Kaliumpermanganat und rührt die Mischung anschließend 16 Stunden bei 50°C bis 60°C. Zur Aufarbeitung gibt man Wasser hinzu, neutralisiert mit wäßriger Natronlauge, zersetzt mit wässriger Natriumhydrogensulfit-Lösung ausgefallenes Mangandioxid, extrahiert anschließend mit Dichlormethan, trocknet die vereinigten organischen Phasen über Natriumsulfat, entfernt das Lösungsmittel unter vermindertem Druck, kristallisiert den Rückstand durch Verrühren mit Ether, saugt ab und trocknet.

Man erhält 4,7 g (40 % der Theorie) an 5-(4-Brombenzylsulfonyl)-3-(4-pyridyl)-1,2,4-thiadiazol vom Schmelzpunkt 173°C.

### Herstellung der Ausgangsverbindungen:

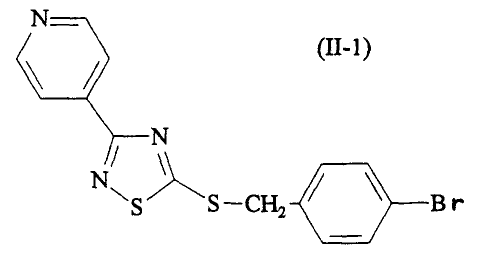

9,8 g (0,05 Mol) 5-Mercapto-3-(4-pyridyl)-1,2,4-thiadiazol werden zusammen mit 13,8 g (0,055 Mol) 4-Brombenzylbromid und 8,3 g (0,06 Mol) Kahumcarbonat in 100 ml Acetonitril für 16 Stunden auf Rückflußtemperatur erhitzt. Zur Aufarbeitung wird im Vakuum eingeengt, der Rückstand in Dichlormethan aufgenommen, mit Wasser gewaschen, über Natriumsulfat getrocknet und abermals im Vakuum eingeengt. Der Rückstand wird durch Verrühren mit Ether kristallisiert, abgesaugt und getrocknet.

Man erhält 7,8 g (43 % der Theorie) an 5-(4-Brombenzylthio)-3-(4-pyridyl)-1,2,4-thiadiazol vom Schmelzpunkt 132°C.

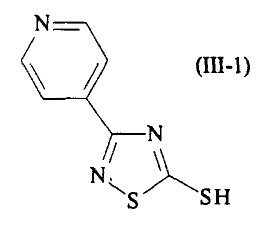

315 g (2,0 Mol) 4-Pyridylamidin-hydrochlorid, 1380 g (6,0 Mol) 30%-ige Natriummethylat-Lösung in Methanol, 380 g (5,0 Mol) Schwefelkohlenstoff und 83,2 g (2,6 Mol) Schwefel-Pulver werden in 2.000 ml Methanol für 16 Stunden auf Rückflußtemperatur erhitzt und anschließend auf Raumtemperatur abgekühlt. Zur Aufarbeitung wird die Reaktionsmischung im Vakuum eingeengt. Der Rückstand wird in heißem Wasser gelöst und filtriert. Das Filtrat wird mit Essigsäure auf pH 4 eingestellt. Ausgefallener Feststoff wird abgesaugt, mit heißem Isopropanol aufgeschlämmt, wieder abgekühlt, abgesaugt und getrocknet.

Man erhält 259 g (66 % der Theorie) an 5-Mercapto-3-(4-pyridyl)-1,2,4-thiadiazol vom Schmelzpunkt 206°C (Zers.).

In entsprechender Weise und gemäß den allgemeinen Angaben zur Herstellung erhält man die folgenden Pyridyl-1,2,4-thiadiazole der Formel (I):

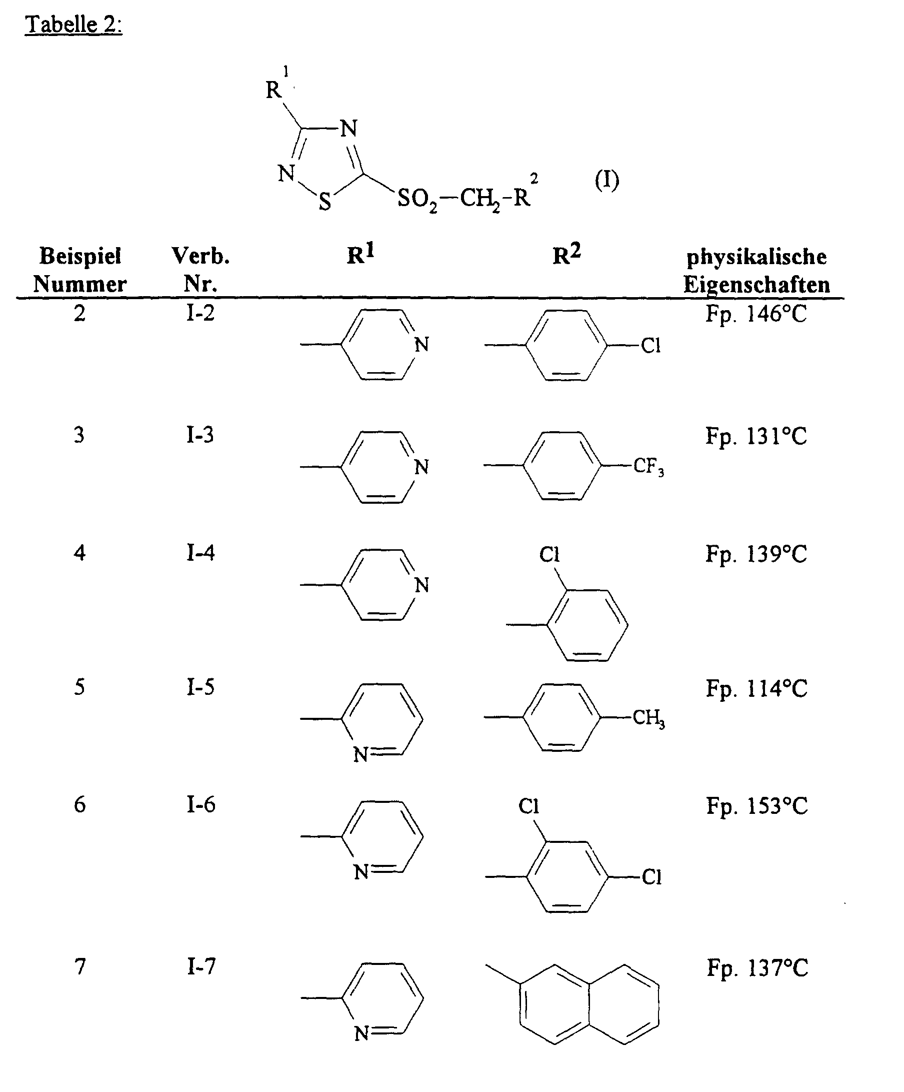

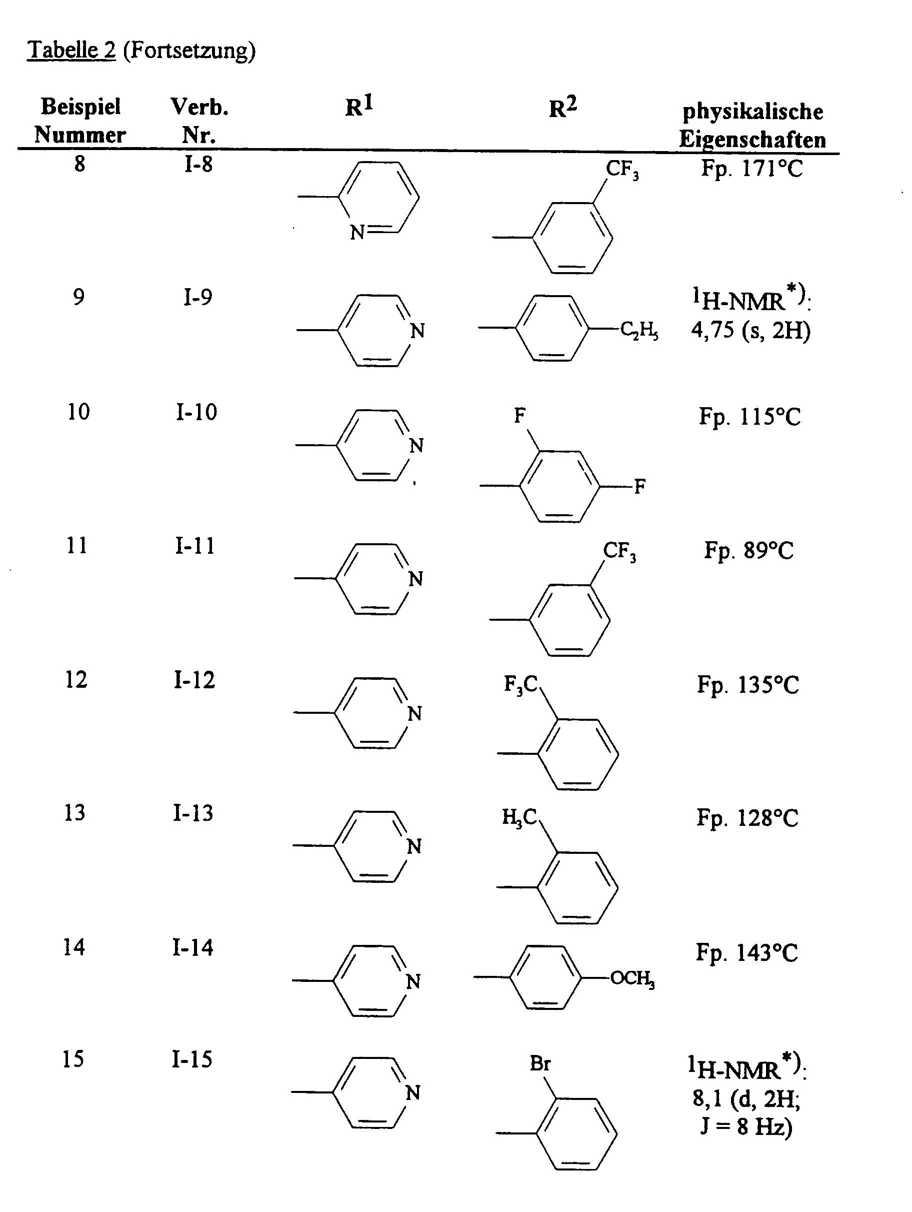

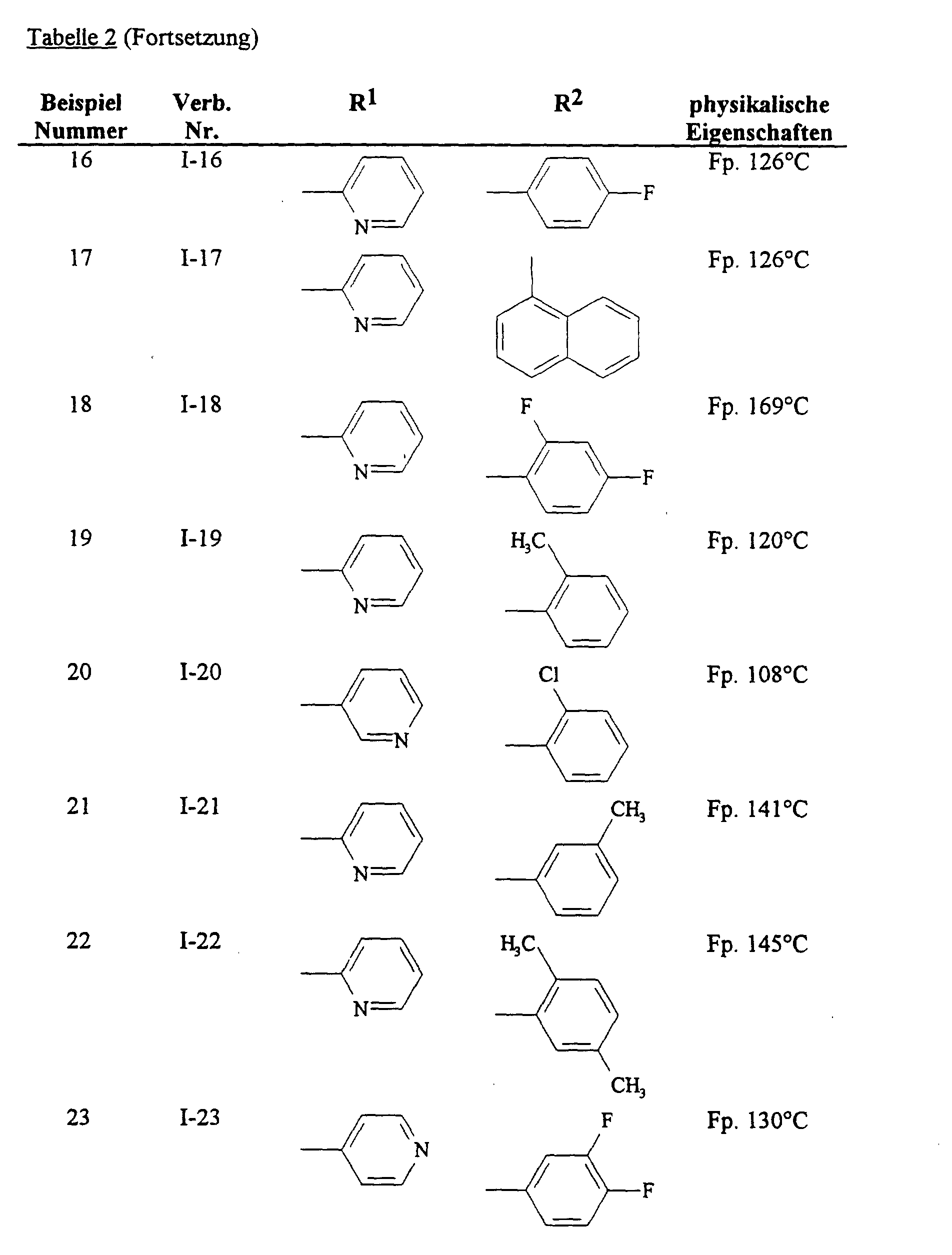

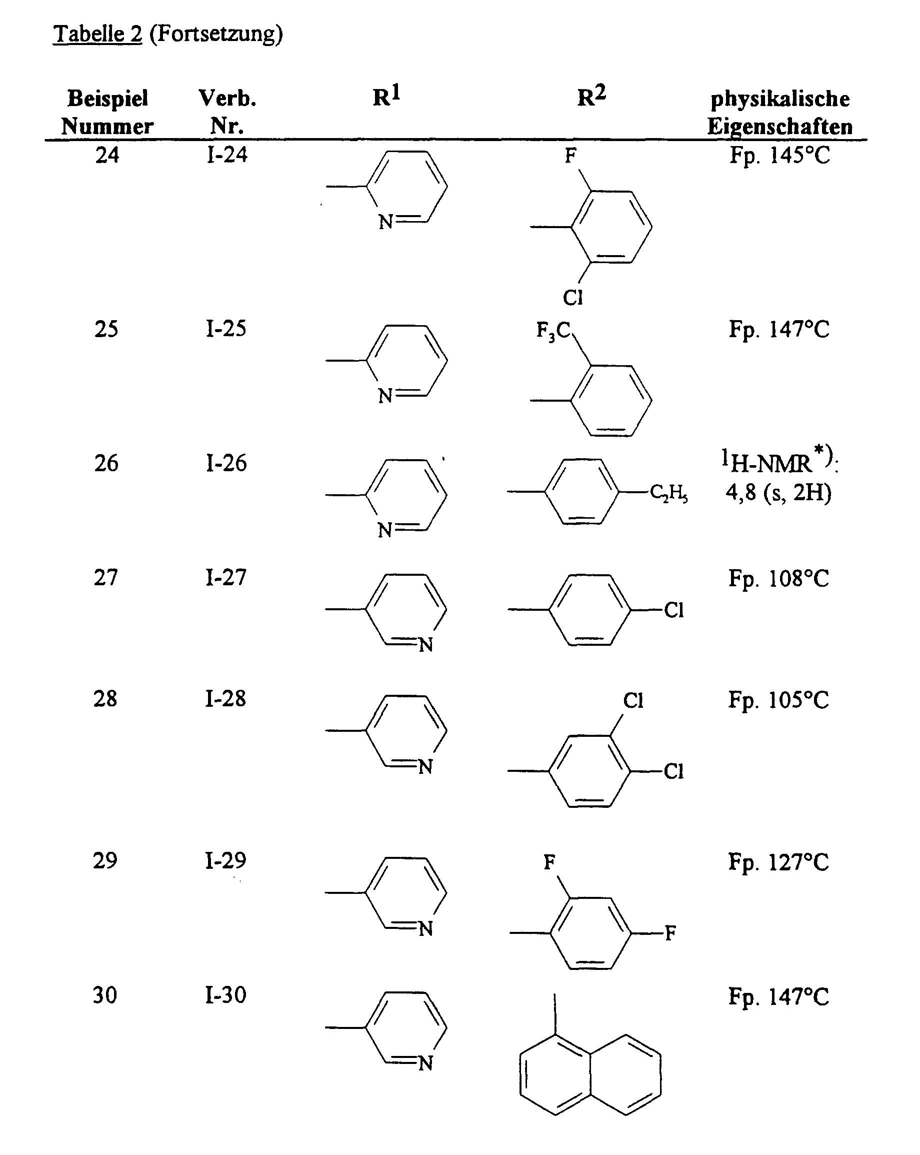

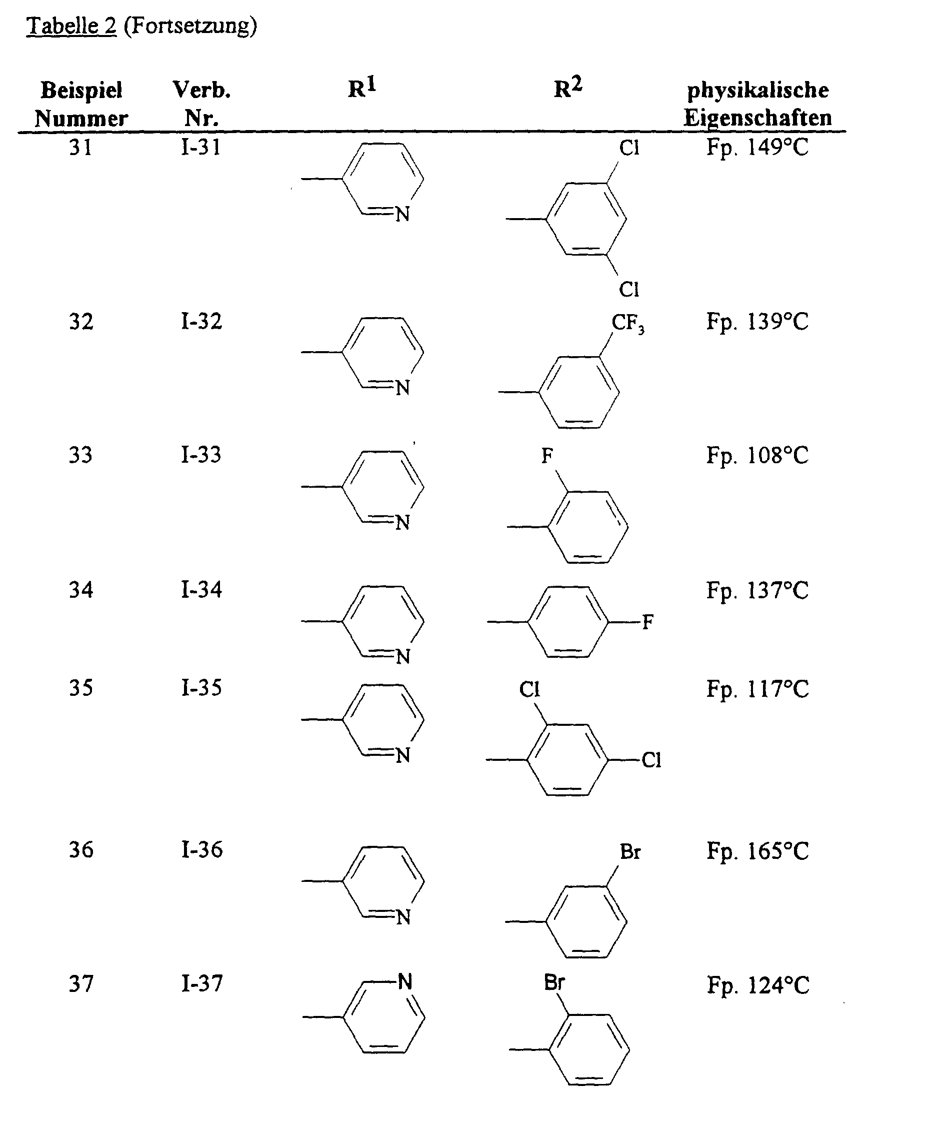

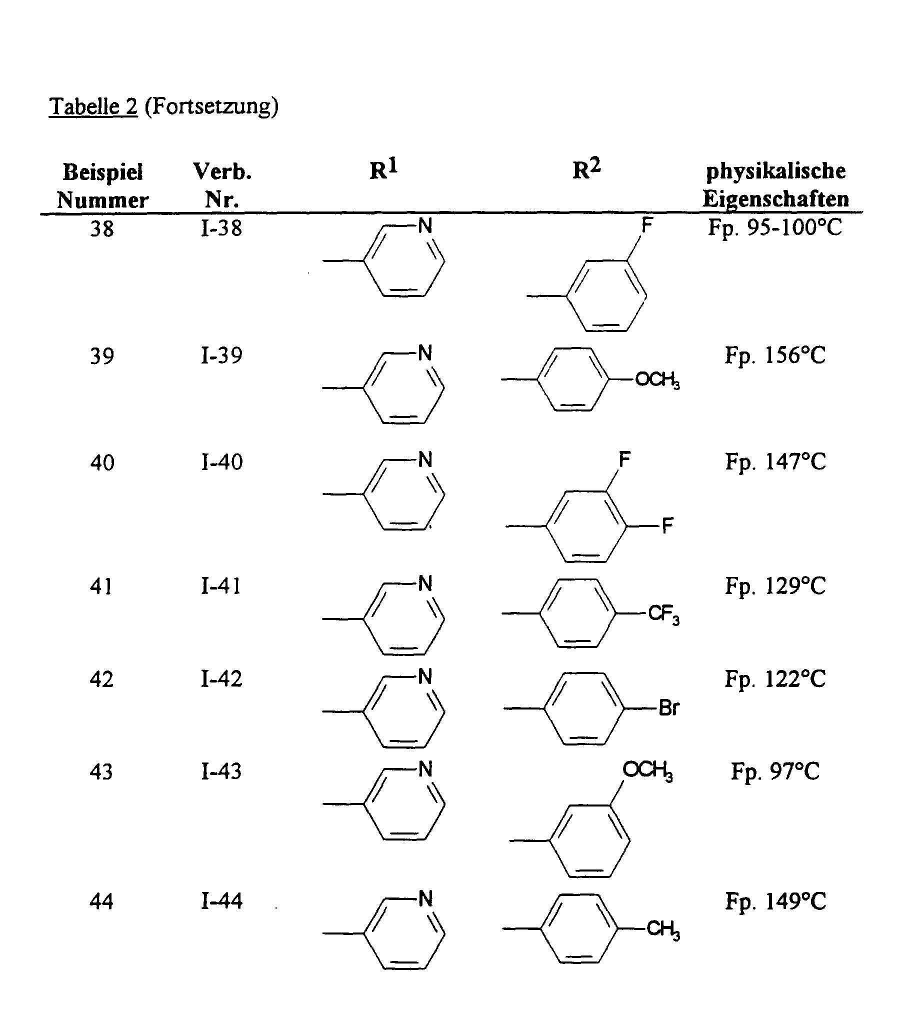

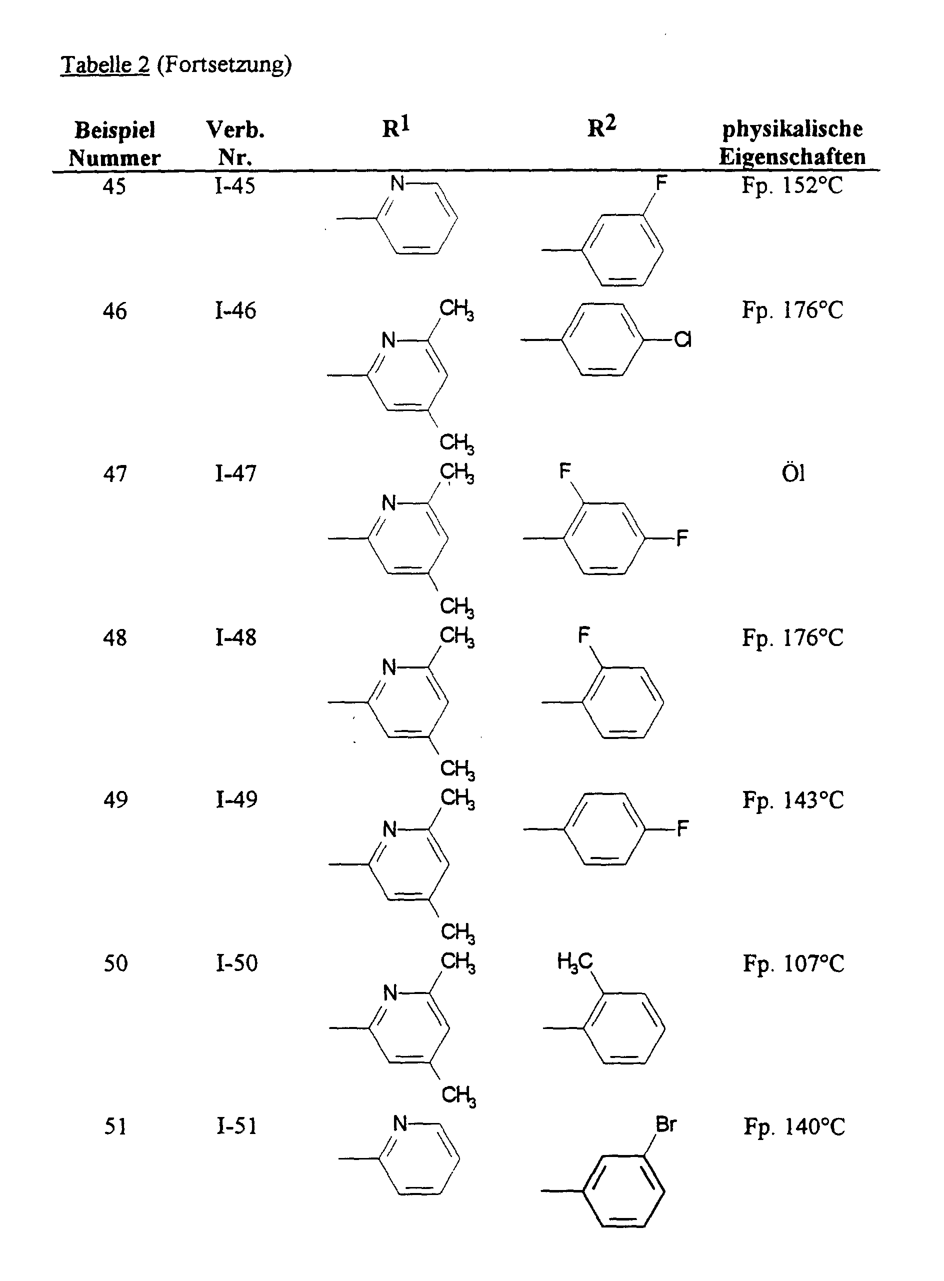

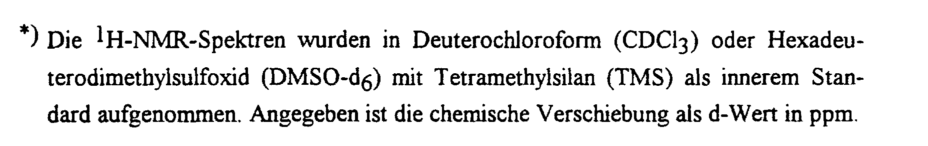

Nach der im Beispiel 1 angegebenen Methode werden auch die in den folgenden Beispielen aufgeführten Ausgangsstoffe der Formel (III) hergestellt.

### Beispiel 52

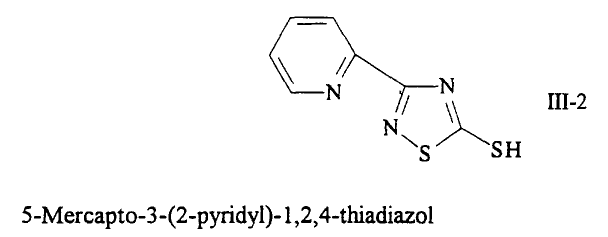

Ausbeute: 178,5 g (46 % der Theorie)
Schmelzpunkt: 197°C

### Beispiel 53

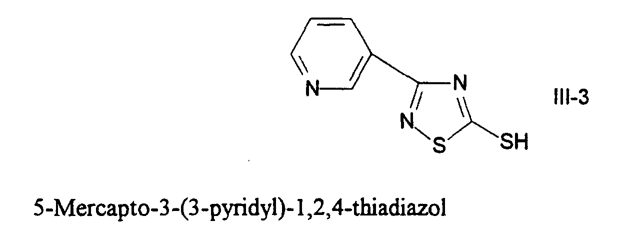

Ausbeute: 289 g (74 % der Theorie)
Schmelzpunkt: 203°C (Zers.)

### Anwendungsbeispiele:

In den folgenden Anwendungsbeispielen wurden die nachstehend aufgeführten Verbindungen als Vergleichssubstanzen eingesetzt:

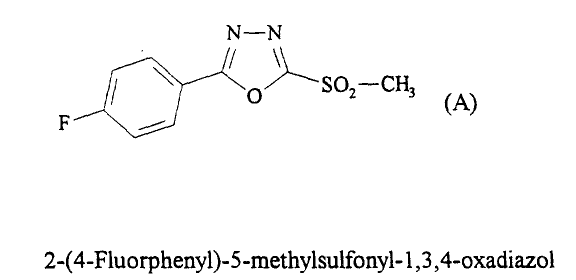

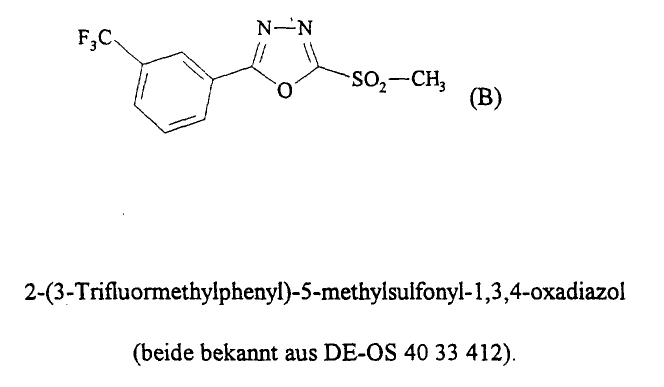

### Beispiel A:

### Venturia-Test (Apfel) / protektiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man ein Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Konidiensuspension des Apfelschorferregers (Venturia inaequalis) inokuliert und verbleiben dann einen Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

In diesem Test zeigen die in den Beispielen 2, 3, 4, 7, 8, 10, 11, 16, 24 und 25 aufgeführten erfindungsgemäßen Stoffe bei einer Wirkstoffkonzentration von 10 ppm in der Spritzflüssigkeit einen Wirkungsgrad von über 70 %, während der Wirkungsgrad der Vergleichssubstanzen (A) und (B) nur 3 bzw. 0 % beträgt.

### Beispiel B:

### Plasmopara-Test (Rebe) / protektiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man ein Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Sporensuspension von Plasmopara viticola inokuliert und verbleiben dann einen Tag in einer Feuchtkammer bei 20°C bis 22°C und 100% relativer Luftfeuchtigkeit. Anschließend werden die Pflanzen 5 Tage im Gewächshaus bei 21°C und 90% Luftfeuchtigkeit aufgestellt. Die Pflanzen werden dann angefeuchtet und einen Tag in eine Feuchtkammer gestellt.

6 Tage nach der Inokulation erfolgt die Auswertung.

In diesem Test zeigen die in den Beispielen 1, 2, 5, 10, 13, 15, 17, 18, 19, 24 und 26 aufgeführten erfindungsgemäßen Stoffe bei einer Wirkstoffkonzentration von 10 ppm in der Spritzflüssigkeit einen Wirkungsgrad von über 80 %, während der Wirkungsgrad der Vergleichssubstanzen (A) und (B) 0 beträgt.

## Patentansprüche

1. Pyridyl-1,2,4-thiadiazole der Formel 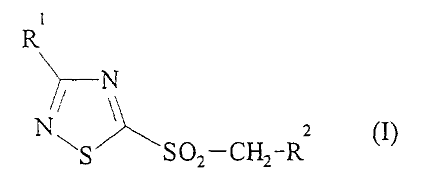 in welcher
R¹ für 2-Pyridyl, 3-Pyridyl oder 4-Pyridyl steht, wobei jeder dieser Reste einfach bis vierfach, gleichartig oder verschieden substituiert sein kann durch Halogen, Cyano, Nitro, jeweils geradkettiges oder verzweigtes Alkyl, Alkoxy oder Alkylsulfonyl mit jeweils 1 bis 6 Kohlenstoffatomen, jeweils geradkettiges oder verzweigtes Halogenalkyl, Halogenalkoxy oder Halogenalkylsulfonyl mit jeweils 1 bis 6 Kohlenstoffatomen und 1 bis 13 gleichen oder verschiedenen Halogenatomen, geradkettiges oder verzweigtes Alkoxycarbonyl mit 1 bis 6 Kohlenstoffatomen in der Alkoxygruppe, geradkettiges oder verzweigtes Alkoximinoalkyl mit 1 bis 6 Kohlenstoffatomen im Alkoxyteil und 1 bis 6 Kohlenstoffatomen im Alkylteil sowie durch Phenyl, welches seinerseits einfach bis vierfach, gleichartig oder verschieden substituiert sein kann durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen, und
R² für Aryl mit 6 bis 10 Kohlenstoffatomen steht, wobei jeder dieser Reste einfach bis fünffach, gleichartig oder verschieden substituiert sein kann durch Halogen, Cyano, Nitro, jeweils geradkettiges oder verzweigtes Alkyl, Alkoxy oder Alkylsulfonyl mit jeweils 1 bis 6 Kohlenstoffatomen, jeweils geradkettiges oder verzweigtes Halogenalkyl, Halogenalkoxy oder Halogenalkylsulfonyl mit jeweils 1 bis 6 Kohlenstoffatomen und 1 bis 13 gleichen oder verschiedenen Halogenatomen, geradkettiges oder verzweigtes Alkoxycarbonyl mit 1 bis 6 Kohlenstoffatomen in der Alkoxygruppe, geradkettiges oder verzweigtes Alkoximinoalkyl mit 1 bis 6 Kohlenstoffatomen im Alkoxyteil und 1 bis 6 Kohlenstoffatomen im Alkylteil sowie durch Phenyl, welches seinerseits einfach bis fünffach, gleichartig oder verschieden substituiert sein kann durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen.

2. Verfahren zur Herstellung von Pyridyl-1,2,4-thiadiazolen der Formel 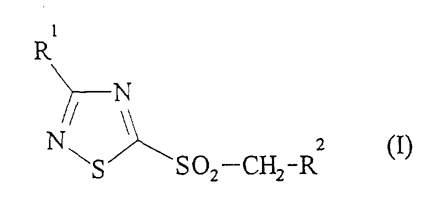 in welcher
R¹ und R² die in Anspruch 1 angegebenen Bedeutungen haben,
**dadurch gekennzeichnet, daß** man 5-Arylalkylthio-1,2,4-thiadiazole der Formel 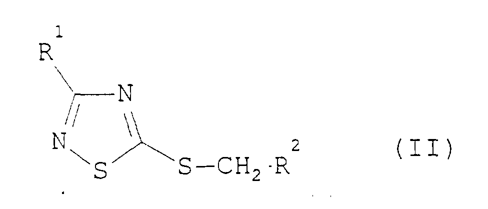 in welcher
R¹ und R² die oben angegebenen Bedeutungen haben,
mit einem Oxidationsmittel gegebenenfalls in Gegenwart eines Verdünnungsmittels und gegebenenfalls in Gegenwart eines Säurebindemittels sowie gegebenenfalls in Gegenwart eines Katalysators umsetzt, und gegebenenfalls anschließend an die so erhaltenen Verbindungen der Formel (I) eine Säure oder ein Metallsalz addiert.

3. Schädlingsbekämpfungsmittel, **gekennzeichnet durch** einen Gehalt an mindestens einem Pyridyl-1,2,4-thiadiazol der Formel (I) gemäß Anspruch 1 bzw. an einem Säureadditions-Salz oder Metallsalz-Komplex eines Pyridyl-1,2,4-thiadiazols der Formel (I).

4. Verwendung von Pyridyl-1,2,4-thiadiazolen der Formel (I) gemäß Anspruch 1 bzw. von deren Säureadditions-Salzen und Metallsalz-Komplexen zur Bekämpfung von Schädlingen

5. Verfahren zur Bekämpfung von Schädlingen, **dadurch gekennzeichnet, daß** man Pyridyl-1,2,4-thiadiazole der Formel (I) gemäß Anspruch 1 bzw. deren Säureadditions-Salze oder Metallsalz-Komplexe auf die Schädlinge und/oder deren Lebensraum ausbringt.

6. Verfahren zur Herstellung von Schädlingsbekämpfungsmitteln, **dadurch gekennzeichnet, daß** man Pyridyl-1,2,4-thiadiazole der Formel (I) gemäß Anspruch 1 bzw. deren Metallsalz-Komplexe oder Säureadditions-Salze mit Streckmitteln und/oder oberflächenaktiven Stoffen vermischt.

7. 5-Arylalkylthio-1,2,4-thiadiazole der Formel 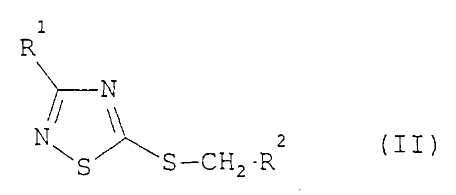 in welcher
R¹ und R² die in Anspruch 1 angegebenen Bedeutungen haben.

8. Verfahren zur Herstellung von 5-Arylalkylthio-1,2,4-thiadiazolen der Formel 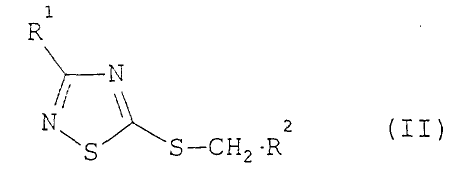 in welcher
R¹ und R² die in Anspruch 1 angegebenen Bedeutungen haben.
**dadurch gekennzeichnet, daß** man 3-Pyridyl-1,2,4-thiadiazolin-5-thione der Formel 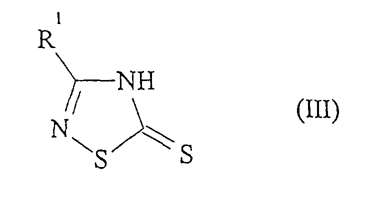 in welcher
R¹ die in Anspruch 1 angegebene Bedeutung hat,
mit Arylalkylhalogeniden der Formel
R²-CH₂-Hal (IV)
in welcher
R² die in Anspruch 1 angegebene Bedeutung hat und
Hal für Halogen steht,
gegebenenfalls in Gegenwart eines Verdünnungsmittels und gegebenenfalls in Gegenwart eines Reaktionshilfsmittels umsetzt.

9. 3-Pyridyl-1,2,4-thiadiazolin-5-thione der Formel 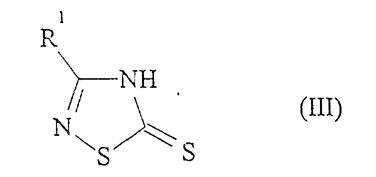 in welcher
R¹ die in Anspruch 1 angegebene Bedeutung hat.

10. Verfahren zur Herstellung von 3-Pyridyl-1,2,4-thiadiazolin-5-thionen der Formel 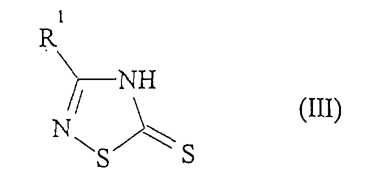 in welcher
R¹ die in Anspruch 1 angegebene hat, **dadurch gekennzeichnet, daß** man Pyridylamidin-hydrochloride der Formel 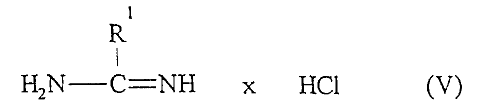
in welcher
R¹ die in Anspruch 1 angegebene Bedeutung hat,
mit Schwefelkohlenstoff in Gegenwart eines Verdünnungsmittels und in Gegenwart eines Reaktionshilfsmittels sowie in Gegenwart eines Katalysators umsetzt.

## Claims

1. Pyridyl-1,2,4-thiadiazoles of the formula 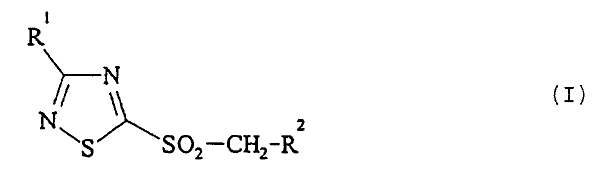 in which
R¹ represents 2-pyridyl, 3-pyridyl or 4-pyridyl, wherein each group may be monosubstituted to tetrasubstituted, identically or differently, by halogen, cyano, nitro or straight-chain or branched alkyl, alkoxy or alkylsulfonyl, each with 1 to 6 carbon atoms, straight-chain or branched haloalkyl, haloalkoxy or haloalkoxysulfonyl, each with 1 to 6 carbon atoms and 1 to 13 identical or different halogen atoms, straight-chain or branched alkoxycarbonyl with 1 to 6 carbon atoms in the alkoxy group, straight-chain or branched alkoximinoalkyl with 1 to 6 carbon atoms in the alkoxy part and 1 to 6 carbon atoms in the alkyl part as well as by phenyl, which in its turn may be monosubstituted to tetrasubstituted, identically or differently, by halogen and/or straight-chain or branched alkyl or alkoxy, each with 1 to 6 carbon atoms, and/or straight chain or branched haloalkyl or haloalkoxy, each with 1 to 6 carbon atoms and 1 to 13 identical or different halogen atoms, and
R² represents aryl with 6 to 10 carbon atoms, wherein each group may be monosubstituted to pentasubstituted, identically or differently, by halogen, cyano, nitro or straight-chain or branched alkyl, alkoxy or alkylsulfonyl, each with 1 to 6 carbon atoms, straight-chain or branched haloalkyl, haloalkoxy or haloalkoxysulfonyl, each with 1 to 6 carbon atoms and 1 to 13 identical or different halogen atoms, straight-chain or branched alkoxycarbonyl with 1 to 6 carbon atoms in the alkoxy group, straight-chain or branched alkoximinoalkyl with 1 to 6 carbon atoms in the alkoxy part and 1 to 6 carbon atoms in the alkyl part as well as by phenyl, which in its turn may be monosubstituted to pentasubstituted, identically or differently, by halogen and/or straight-chain or branched alkyl or alkoxy, each with 1 to 6 carbon atoms, and/or straight chain or branched haloalkyl or haloalkoxy, each with 1 to 6 carbon atoms and 1 to 13 identical or different halogen atoms.

2. A process for preparing pyridyl-1,2,4-thidiazoles of the formula 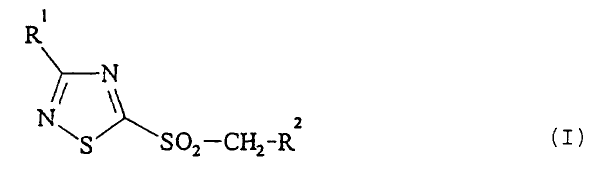 in which
R¹ and R² are defined in the same way as in Claim 1,
**characterised in that** 5-arylalkylthio-1,2,4-thiadiazoles of the formula 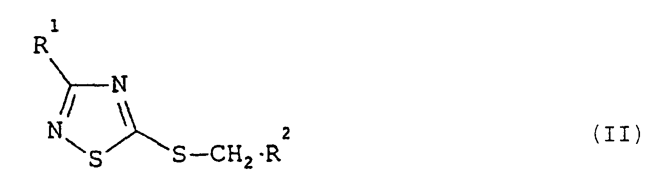 in which
R¹ and R² are defined in the same way as above,
are reacted with an oxidising agent, optionally in the presence of a diluent and optionally in the presence of an acid binder and also optionally in the presence of a catalyst, and that an acid or a metal salt is then optionally added to the compounds of the formula (I) obtained in this way.

3. A pesticide, **characterised by** a concentration of at least one pyridyl-1,2,4-thiadiazole of the formula (I) in accordance with Claim 1 or of an acid addition salt or metal salt complex of a pyridyl-1,2,4-thiadiazole of the formula (I).

4. The use of pyridyl-1,2,4-thiadiazoles of the formula (I) in accordance with Claim 1 or of their acid addition salts and metal salt complexes for controlling pests.

5. A process for controlling pests, **characterised in that** pyridyl-1,2,4-thiadiazoles of the formula (I) in accordance with Claim 1 or their acid addition salts or metal salt complexes are applied to the pests and/or their habitat.

6. A process for preparing pesticides, **characterised in that** pyridyl-1,2,4-thiadiazoles of the formula (I) in accordance with Claim 1 or their metal salt complexes or acid addition salts are blended with extenders and/or surface-active substances.

7. 5-arylalkylthio-1,2,4-thiadiazoles of the formula 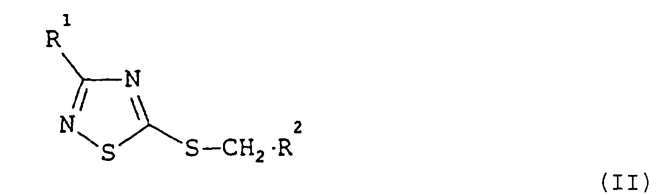 in which
R¹ and R² are defined in the same way as in Claim 1.

8. A process for preparing 5-arylalkylthio-1,2,4-thiadiazoles of the formula 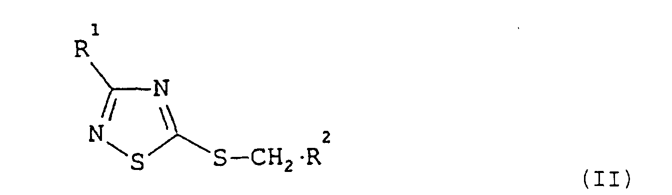 in which
R¹ and R² are defined in the same way as in Claim 1
**characterised in that**, 3-pyridyl-1,2,4-thiadiazoline-5-thiones of the formula 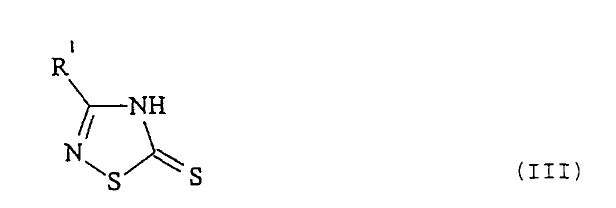 in which
R¹ is defined in the same way as in Claim 1,
are reacted with arylalkyl halides of the formula
R²-CH₂-Hal (IV)
in which
R² is defined in the same way as in Claim 1 and
Hal represents a halogen,
optionally in the presence of a diluent and optionally in the presence of a reaction auxiliary substance.

9. 3-pyridyl-1,2,4-thiadiazoline-5-thiones of the formula 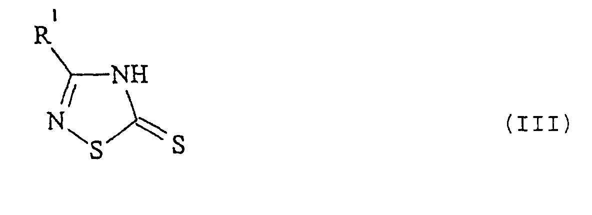 in which
R¹ is defined in the same way as in Claim 1.

10. A process for preparing 3-pyridyl-1,2,4-thiadiazoline-5-thiones of the formula 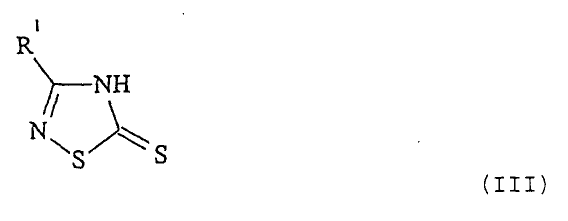 in which
R¹ is defined in the same way as in Claim 1,
**characterised in that** pyridylamidine hydrochlorides of the formula 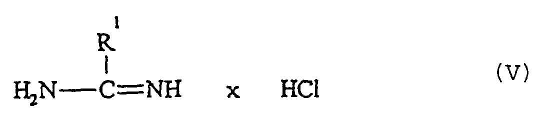 in which
R¹ is defined in the same way as in Claim 1,
are reacted with carbon disulfide in the presence of a diluent and in the presence of a reaction auxiliary substance and also in the presence of a catalyst.

## Revendications

1. Pyridyl-1,2,4-thiadiazoles de formule 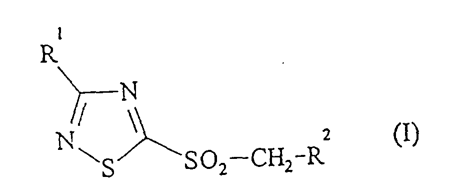 dans laquelle
R¹ est un reste 2-pyridyle, 3-pyridyle ou 4-pyridyle, chacun de ces restes pouvant être substitué une à quatre fois identiques ou différentes par un halogène, un radical cyano, nitro, un radical alkyle, alkoxy ou alkylsulfonyle chacun linéaire ou ramifié, avec chacun 1 à 6 atomes de carbone, un radical halogénalkyle, halogénalkoxy ou halogénalkylsulfonyle chacun linéaire ou ramifié, avec chacun 1 à 6 atomes de carbone et 1 à 13 atomes d'halogènes identiques ou différents, un radical alkoxycarbonyle linéaire ou ramifié ayant 1 à 6 atomes de carbone dans le groupe alkoxy, un radical alkoximino-alkyle linéaire ou ramifié ayant 1 à 6 atomes de carbone dans la partie alkoxy et 1 à 6 atomes de carbone dans la partie alkyle ainsi qu'un radical phényle qui peut être substitué de son côté une à quatre fois identiques ou différentes par un halogène et/ou un radical alkyle ou un radical alkoxy linéaire ou ramifié ayant chacun 1 à 6 atomes de carbone et/ou un radical halogénalkyle ou un radical halogénalkoxy linéaire ou ramifié ayant chacun 1 à 6 atomes de carbone et 1 à 13 atomes d'halogènes identiques ou différents, et
R² est un reste aryle ayant 6 à 10 atomes de carbone, chacun de ces restes aryle pouvant être substitué une à cinq fois identiques ou différentes par un radical halogéno, cyano, nitro, un radical alkyle, alkoxy ou alkylsulfonyle chacun linéaire ou ramifié ayant chacun 1 à 6 atomes de carbone, un radical halogénalkyle, halogénalkoxy ou halogénalkylsulfonyle chacun linéaire ou ramifié, ayant chacun 1 à 6 atomes de carbone et 1 à 13 atomes d'halogènes identiques ou différents, un radical alkoxycarbonyle linéaire ou ramifié ayant 1 à 6 atomes de carbone dans le groupe alkoxy, un radical alkoximino-alkyle linéaire ou ramifié ayant 1 à 6 atomes de carbone dans la partie alkoxy et 1 à 6 atomes de carbone dans la partie alkyle, ainsi que par un radical phényle qui peut être substitué de son côté une à cinq fois identiques ou différentes par un halogène et/ou par un radical alkyle ou alkoxy linéaire ou ramifié ayant chacun 1 à 6 atomes de carbone et/ou par un radical halogénalkyle ou halogénalkoxy linéaire ou ramifié ayant chacun 1 à 6 atomes de carbone et 1 à 13 atomes d'halogènes identiques ou différents.

2. Procédé de production de pyridyl-1,2,4-thiadiazoles de formule 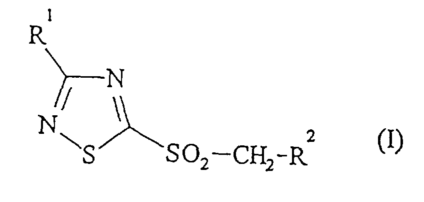 dans laquelle
R¹ et R² ont les définitions indiquées dans la revendication 1,
**caractérisé en ce qu'**on fait réagir des 5-arylalkylthio-1,2,4-thiadiazoles de formule 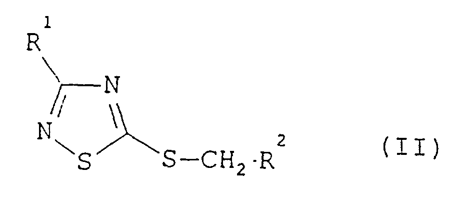 dans laquelle
R¹ et R² ont les définitions indiquées ci-dessus,
avec un agent oxydant, éventuellement en présence d'un diluant et en présence éventuelle d'un accepteur d'acide ainsi que, le cas échéant, en présence d'un catalyseur, et on additionne ensuite éventuellement sur les composés de formule (I) ainsi obtenus un acide ou un sel métallique.

3. Composition pesticide, **caractérisée par** une teneur en au moins un pyridyl-1,2,4-thiadiazole de formule (I) suivant la revendication 1, ou en un sel d'addition d'acide ou un complexe de sel métallique d'un pyridyl-1,2,4-thiadiazole de formule (I).

4. Utilisation de pyridyl-1,2,4-thiadiazoles de formule (I) suivant la revendication 1 ou de leurs sels d'addition d'acides et de leurs complexes de sels métalliques pour combattre des parasites.

5. Procédé pour combattre des parasites, **caractérisé en ce qu'**on fait agir des pyridyl-1,2,4-thiadiazoles de formule (I) suivant la revendication 1 ou leurs sels d'addition d'acides ou leurs complexes de sels métalliques sur les parasites et/ou sur leur milieu.

6. Procédé de préparation de compositions pesticides,
**caractérisé en ce qu'**on mélange des pyridyl-1,2,4-thiadiazoles de formule (I) suivant la revendication 1 ou leurs complexes de sels métalliques ou leurs sels d'addition d'acides avec des diluants et/ou des agents tensio-actifs.

7. 5-arylalkylthio-1,2,4-thiadiazoles de formule 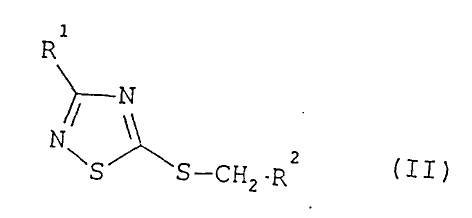 dans laquelle
R¹ et R² ont les définitions indiquées dans la revendication 1.

8. Procédé de production de 5-arylalkylthio-1,2,4-thiadiazoles de formule 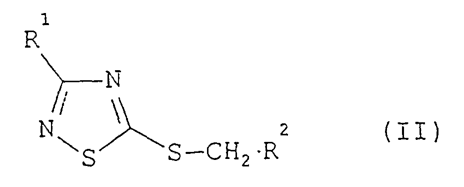 dans laquelle
R¹ et R² ont les définitions indiquées dans la revendication 1,
**caractérisé en ce qu'**on fait réagir des 3-pyridyl-1,2,4-thiadiazoline-5-thiones de formule 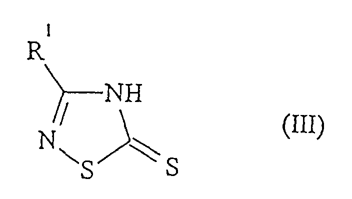 dans laquelle
R¹ a la définition indiquée dans la revendication 1,
avec des halogénures d'arylalkyle de formule
R²-CH₂-Hal (IV)
dans laquelle
R² a la définition indiquée dans la revendication 1 et
Hal représente un halogène,
le cas échéant, en présence d'un diluant et en présence éventuelle d'un auxiliaire de réaction.

9. 3-pyridyl-1,2,4-thiadiazoline-5-thiones de formule 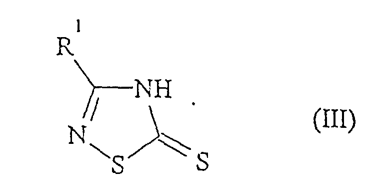 dans laquelle
R¹ a la définition indiquée dans la revendication 1.

10. Procédé de production de 3-pyridyl-1,2,4-thiadiazoline-5-thiones de formule 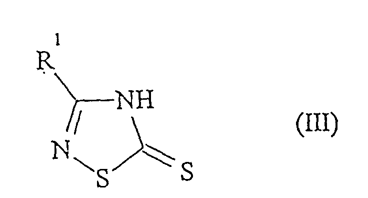 dans laquelle
R¹ a la définition indiquée dans la revendication 1,
**caractérisé en ce qu'**on fait réagir des chlorhydrates de pyridylamidine de formule 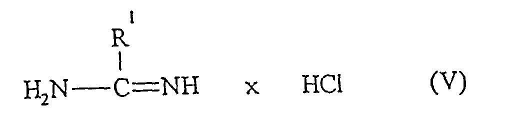 dans laquelle
R¹ a la définition indiquée dans la revendication 1,
avec le sulfure de carbone en présence d'un diluant et en présence d'un auxiliaire de réaction ainsi qu'en présence d'un catalyseur.
